# EUROPEAN PATENT APPLICATION

(11) **EP 4 095 450 A1**
(43) Date of publication of application: **30.11.2022**
(21) Application number: 20920885.9
(22) Date of filing: 07.10.2020
(51) Int. Cl.: F24F 3/16, F24F 13/20, F24F 13/28, F24F 13/08, F24F 11/56, F24F 11/88, F24F 11/64, F24F 11/70

(54) **AIR PURIFIER**

(30) Priority: 27.02.2020 KR 20200024321
(71) Applicant: APC TEC. Co., Ltd., Chungcheongbuk-do 27462 (KR)
(72) Inventor: KIM, Seung Jin, Seoul 05042 (KR)
(74) Representative: BCKIP Part mbB
(86) International application number: PCT/KR2020/013641
(87) International publication number: WO 2021/172683

(57) **Abstract**

The present invention relates to an air cleaner, and more specifically, to an air cleaner in which a filter part using a photocatalyst is formed in a module type to facilitate replacement and cleaning and which includes an insert fan (100) which is disposed inward from a suction port of a casing forming an exterior and including the suction port disposed at one side and a discharge port disposed at the other side, and suctions ambient air, a driving motor (200) provided to be axially detachably coupled to the insert fan (100) in a direction from the discharge port, a suction unit fixing part (300) in which an accommodation space (310), into which the insert fan (100) and the driving motor (200) are fixedly inserted in the direction from the suction port toward the discharge port and which is finished by inserting a cover member (400) in the direction from the discharge port, the cover member (400) in which a plurality of protruding fixing members (410) are disposed on a side surface of the suction unit fixing part (300) to fix the driving motor (200) in the direction from the discharge port and a plurality of first ultraviolet (UV) lamps (420) are disposed to provide light emission toward the suction port, a filter part (500) which is provided to be pressed against and coupled to the cover member (400) in the direction from the discharge port and purifies air discharged by the insert fan (100), a pollution information collection unit (600) disposed in or around the casing to collect information about surrounding air, and a control unit (700) which transmits pollution information collected by the pollution information collection unit (600) to an outside through a communication module (710) and controls the driving motor (200), the first UV lamps (420), and the filter part (500) using a control signal received from an external device or an input control signal to reduce air pollution through Internet of Things (IoT)-based technology through which data communication is facilitated.

## Description

### BACKGROUND

### 1. Field of the Invention

The present invention relates to an air cleaner, and more specifically, to an air cleaner in which a filter part using a photocatalyst is formed in a module type to facilitate replacement and cleaning, and which includes an insert fan, a suction unit fixing part, a cover member, the filter part including the photocatalyst, and an Internet of Things (IoT)-based control unit to reduce air pollution through IoT-based technology through which data communication with an external device is facilitated.

### 2. Discussion of Related Art

Although clean air, uniform temperature, and proper humidity are major factors for determining the quality of an indoor environment, various types of harmful substances are contained in indoor or closed spaces due to industrialization and urbanization, and thus the health of many people is threatened by spending time exposed to these harmful substances.

For example, infectious microorganisms (staphylococcus aureus, legionella, tuberculosis bacilli, aspergillus, influenza germs, polio viruses, foot-and-mouth disease viruses, and the like) that are spread through air, organic pollutants (combustion compounds, phenolic compounds, environmental hormones, and the like), atmospheric pollutants (automobile exhaust gas, nitrogen oxides, sulfur oxides, and dioxins which are discharged from incinerators, and the like), and hazardous odors (formaldehyde, acetaldehyde, xylene, toluene, styrene, and the like) are contained in indoor air, are accumulated in or directly affect the human body to weaken immunity, cause various diseases such as chronic bronchitis, lung function damage, and various infectious diseases, and directly threaten life.

Various methods have been attempted to improve the quality of indoor air in such a harmful environment, and air cleaning using a photocatalyst is also one method.

An air cleaning method using a photocatalyst is a method using strong sterilization and oxidation reactions of a titanium dioxide (TiO₂), which is mainly used as a photocatalyst, and has characteristics of showing strong air purification effects in which oxidation-reduction reactions and hydrophilic reactions occur at the same time within molecules when ultraviolet light of 380 nm or less is emitted to nanometer-sized titanium dioxide (TiO₂) crystals, and an effect like that of incineration occurs on the surface of the photocatalyst at an absolute temperature of 30,000 °K to oxidize and decompose volatile organic compounds (VOCs) such as acetaldehyde, xylene, toluene, and styrene, harmful substances generated in buildings such as radon gas and formaldehyde, odorous gases such as hydrogen sulfide and ammonia, atmospheric pollutants such as sulfur oxides (SOₓ) and nitrogen oxides (NOₓ), organic chlorine compounds such as other trichlorethylenes, phenol compounds, polyvinylchloide (PVC), environmental hormones (bisphenol, nonylphenol, and estradiol), dioxin acetaldehyde, xylene, toluene, styrene hydrogen sulfide, methyl mercaptan, emulsified methyl, trimethylamine, an isovaleric acid, and ammonia, to decompose microorganisms such as bacteria to oxidize into CO₂ and H₂O with strong sterilization power, and to remove toxic substances in the air.

In an air cleaner using the sterilization and oxidation reactions of the photocatalyst, since the photocatalysis occurs on a surface of the photocatalyst with which a base material is coated, as the contact area with air increases, the treatment capacity increases, and the treatment capacity increases by properly emitting light energy corresponding to band-gab energy (Eg) of the titanium dioxide.

In addition, since the sterilization and oxidation reactions by the photocatalyst occur due to contact at the surface of the photocatalyst, a cleanliness state of the surface of the photocatalyst is a factor for determining air purification efficiency, and since the photocatalyst has a super hydrophilic property by absorbing ultraviolet light, when in contact with moisture, a water film is formed on the surface of the photocatalyst to perform self-purification for removing foreign substances, but the water film blocks contact between the photocatalyst and air (pollutants) to reduce the purification efficiency.

In this case, an amount of water generated through an oxidation process is very small, and the water evaporates in the form of vapor at the same time as the reaction, and thus cannot be used for self-purification.

Theoretically, since a catalyst is not changed even when causing a chemical reaction, the lifetime of the catalyst is permanent. However, in fact, the catalyst has a characteristic that the activity of the catalyst decreases because the surface of the catalyst on which the chemical reaction occurs is contaminated, and in order to solve this problem, the surface should be washed and recycled.

However, in order to wash a filter member including such a photocatalyst, a washing part should be included in the air cleaner, or a filter member should be withdrawn from the air cleaner. When the washing part is included, there is a problem that the structure of the air cleaner is complicated. In addition, it is difficult to check the replacement period of the filter member.

For this region, when ions which belong to alkalis such as calcium (Ca) and magnesium (Mg) tend to be adsorbed on a surface and deposited, and the surface of the photocatalyst is contaminated by adhesion of organic matter, there is no problem of decomposing the organic matter using the photocatalytic reaction, but in the case of contamination of the surface of the photocatalyst due to inorganic substances, the inorganic substances are not decomposed by the photocatalytic reaction, and thus remain on the surface of the photocatalyst.

Although the air purification using the photocatalyst can achieve an efficient air purification effect by fully reflecting such characteristics of the photocatalyst, the conventionally proposed various types of products do not reflect the characteristics of the photocatalyst, and as a result, there is a problem of limiting the air cleaning ability of the photocatalyst.

Particularly, since a nitrogen oxide (NOₓ) and a sulfur oxide (SOₓ) are changed into a low-concentration nitric acid or sulfuric acid, and the low-concentration nitric acid or sulfuric acid from a permeation film remains on the surface of the photocatalyst and degrades the photocatalytic action during an oxidation process, the permeation film needs to be removed through regular cleaning. In the conventional air purifier using the photocatalyst, such a problem is solved through a method of separating a photocatalyst filter from a main body and washing the photocatalyst filter with water about once a year, but, since detaching the photocatalyst filter for washing may be cumbersome, or the photocatalyst filter can be contaminated in a short time according to an environment, it is actually difficult to expect a continuous catalytic effect.

### [Related Art Document]

### [Patent Document]

(Patent Document 1) Korean Patent Registration No. 10-0235088 (Registered on September 21, 1999).

### SUMMARY OF THE INVENTION

The present invention is directed to providing an air cleaner in which a filter member is provided in a module type to be easily replaced along with a photocatalytic reaction unit.

The present invention is also directed to providing the air cleaner which suctions air using an insert fan (100) which is disposed inward from a suction port of a casing forming an exterior and including the suction port disposed at one side and a discharge port disposed at the other side, a driving motor (200) provided to be axially detachably coupled to the insert fan (100) in a direction from the discharge port, and a suction unit fixing part (300) in which an accommodation space (310), into which the insert fan (100) and the driving motor (200) are fixedly inserted in the direction from the suction port toward the discharge port and which is finished by inserting a cover member (400) in the direction from the discharge port.

The present invention is also directed to providing the air cleaner in which the driving motor is stably fixed and a direction, in which light is emitted, is set to facilitate filtering through the cover member (400) which is provided so that a plurality of protruding fixing members (410) are disposed on a side surface of the suction unit fixing part (300) to fix the driving motor (200) in the direction from the discharge port, and a plurality of first ultraviolet (UV) lamps (420) are disposed to provide light emission toward the suction port.

The present invention is also directed to providing the air cleaner which purifies polluted air, collects pollution information, and is easily controlled according to a degree of pollution and which includes a filter part (500) which is provided to be pressed against and coupled to the cover member (400) in the direction from the discharge port and purifies air discharged by the insert fan (100), a pollution information collection unit (600), and a control unit (700) which transmits pollution information collected by the pollution information collection unit (600) to an external device through a communication module (710) and controls the driving motor (200), the first UV lamps (420), and the filter part (500) using a control signal received from an external device or an input control signal.

The present invention is also directed to providing the air cleaner in which a support force of a cover attached to a filter is improved, and air easily flows by sequentially arranging a filter front cover (510), a filter member (520), and a filter rear cover (530) in a direction from the insert fans (100) toward the discharge port, wherein the filter front cover (510) is provided with a first edge (511) having a circular shape and a protruding fixing member (512) having a circular shape and disposed at an inner center of the first edge (511), first support ribs (513) radially formed between the first edge (511) and the protruding fixing member (512), a plurality of first air through holes (514) formed between the first support ribs (513), and a plurality of first fixing through holes (515) disposed in the first support rib (513) to fix the filter front cover (510) and the filter rear cover (530).

The present invention is also directed to providing the air cleaner in which a support force is improved and air circulation is easily accomplished by forming a second edge (531) having the same size and the same shape as the first edge (511), an accommodation edge (532) into which the protruding fixing member (512) is fixedly inserted at a central position, and a second support rib (533) and a plurality of second air through holes (534) between the second edge (531) and the accommodation edge (532), and further providing second fixing through holes (535) at positions corresponding to the first fixing through holes (515).

The present invention is also directed to providing the air cleaner which decomposes pollutants using a photocatalyst and to which a UV lamp is easily fixed by further including a second UV lamp (540) and a photocatalyst (550) between the filter front cover (510) and the filter member (520), wherein a lamp support (541) having a lamp shade (542) to maintain an angle at which light is emitted in a direction from the filter front cover (510) toward the filter member (520) is provided, the second UV lamp (540) is fixed to the lamp support (541), lengths of left and right sides of the lamp shade (542) are asymmetrically formed to be different, and one side having a greater length is formed as a shade space part (543) so that UV light being emitted is directly emitted to the filter member (520).

The present invention is also directed to providing the air cleaner which decomposes harmful substances through an oxidation reaction by coating a surface of the photocatalyst (550) with a photocatalyst agent, wherein the base material is prepared by mixing and grinding a phosphorescent material at 37 wt%, a zeolite at 37 wt%, a binder (sodium silicate) at 25 wt%, a titanium dioxide at 1 wt%, and ceramic balls to prepare a base powder material, applying moisture to the prepared base powder material to prepare the base powder material in a dough state, and balling and drying the base powder material in the dough state, and the photocatalyst agent is an agent in which any one oxide among a titanium dioxide (TiO₂), a zirconia (ZrO₂), a zinc oxide (ZnO), and an iron dioxide (Fe₂O₃) is dispersed in a solvent.

The present invention is also directed to providing the air cleaner in which an air flow is controlled to facilitate filtering by providing a pair of left and right insert fans (100), a pair of left and right driving motors (200), and a pair of left and right filter parts (500) each provided as one group, wherein the accommodation space (310) is disposed to be divided into left and right spaces side by side so that the suction unit fixing part (300) accommodates the insert fans (100) and the driving motors (200) each disposed in the pair side by side, and the suction unit fixing part (300) further includes an air guide formed spirally in a rotation direction of the insert fan (100) and the driving motor (200) therein.

The present invention is also directed to providing the air cleaner which controls a driving force according to a degree of pollution by further including an internal memory (720) in the control unit (700) and including at least any one among a fine dust sensor, a CO₂ sensor, and a volatile organic compounds (VOC) sensor in the pollution information collection unit (600), wherein a signal processor (730), which compares a pollution critical value stored in the internal memory (720) and pollution information collected by the pollution information collection unit (600) so as to control a rotation speed and a rotation time of the driving motors (200), is further provided, and the collected pollution information versus driving information of the driving motor (200) are mapped and stored in the internal memory (720) in advance.

The present invention is also directed to providing the air cleaner which is easily controlled from the outside through a communication module by generating, by the signal processor (730), a pollution trigger to drive a contamination processor when receiving the pollution information from the pollution information collection unit (600), generating, by the signal processor (730), a control trigger to drive a control processor when receiving a control signal through the communication module (710), wherein the contamination processor and the control processor are provided to determine turning ON/OFF, a rotation speed, or a rotation time of the driving motor (200) according to priority information preset in the internal memory (720), and transmitting determined information to an external device through the communication module (710) according to the information determined by the control unit (700).

Meanwhile, objectives to be solved through the present invention are not limited to the above-described objectives, and other objectives which are not described above will be clearly understood by those skilled in the art through the following specification.

According to an aspect of the present invention, there is provided an air cleaner including an insert fan (100) which is disposed inward from a suction port of a casing forming an exterior and including the suction port disposed at one side and a discharge port disposed at the other side, and suctions ambient air.

The air cleaner may include a driving motor (200) provided to be axially detachably coupled to the insert fan (100) in a direction from the discharge port.

The air cleaner may include a suction unit fixing part (300) in which an accommodation space (310), into which the insert fan (100) and the driving motor (200) are fixedly inserted in the direction from the suction port toward the discharge port and which is finished by inserting a cover member (400) in the direction from the discharge port.

The air cleaner may include the cover member (400) in which a plurality of protruding fixing members (410) are disposed on a side surface of the suction unit fixing part (300) to fix the driving motor (200) in the direction from the discharge port and a plurality of first UV lamps (420) are disposed to provide light emission toward the suction port.

The air cleaner may include a filter part (500) which is provided to be pressed against and coupled to the cover member (400) in the direction from the discharge port and purifies air discharged by the insert fan (100).

The air cleaner may include a pollution information collection unit (600).

The air cleaner may include a control unit (700) which transmits pollution information collected by the pollution information collection unit (600) to an external device through a communication module (710), and controls the driving motor (200), the first UV lamps (420), and the filter part (500) using a control signal received from an external device or an input control signal.

In the filter part (500), a filter front cover (510), a filter member (520), and a filter rear cover (530) may be sequentially disposed in a direction from the insert fan (100) toward the discharge port, wherein the filter front cover (510) may be provided with a first edge (511) having a circular shape and a protruding fixing member (512) having a circular shape and disposed at an inner center of the first edge (511), first support ribs (513) radially formed between the first edge (511) and the protruding fixing member (512), a plurality of first air through holes (514) formed between the first support ribs (513), and a plurality of first fixing through holes (515) disposed in the first support rib (513) to fix the filter front cover (510) and the filter rear cover (530).

The filter rear cover (530) may be provided with a second edge (531 having the same size and the same shape as the first edge (511), an accommodation edge (532), into which the protruding fixing member (512) is fixedly inserted, formed at a central position of the filter rear cover (530), a second support rib (533) and a plurality of second air through holes (534) formed between the second edge (531) and the accommodation edge (532), and second fixing through holes (535) further provided at positions corresponding to the first fixing through holes (515).

The filter part (500) may further include a second UV lamp (540) and a photocatalyst (550) between the filter front cover (510) and the filter member (520), wherein the second UV lamp (540) may include a lamp support (541) having a lamp shade (542) to maintain an angle at which light is emitted from the filter front cover (510) toward the filter member (520), the second UV lamp (540) may be fixed to the lamp support (541), lengths of left and right sides of the lamp shade (542) may be asymmetrically formed to be different, one side having a greater length may be formed as a shade space part (543), and UV light being emitted may be directly emitted to the filter member (520).

The photocatalyst (550) may be formed by coating a surface of a base material having a spherical shape with a photocatalyst agent, wherein the base material may prepared by mixing and grinding a phosphorescent material at 37 wt%, a zeolite at 37 wt%, a binder (sodium silicate) at 25 wt%, a titanium dioxide at 1 wt%, and ceramic balls to prepare a base powder material, applying moisture to the prepared base powder material to prepare the base powder material in a dough state, and balling and drying the base powder material in the dough state, and the photocatalyst agent may be an agent in which any one oxide among a titanium dioxide (TiO₂), a zirconia (ZrO₂), a zinc oxide (ZnO), an iron dioxide (Fe₂O₃) is dispersed in a solvent.

A pair of left and right insert fans (100), a pair of left and right driving motors (200), and a pair of left and right filter parts (500) each may be provided as one group, the accommodation space (310) may be disposed to be divided into left and right spaces side by side so that the suction unit fixing part (300) may accommodate the insert fans (100) and the driving motors (200) each disposed in the pair side by side, and the suction unit fixing part (300) may further include an air guide formed spirally in a rotation direction of the insert fans (100) and the driving motors (200) therein.

The control unit (700) may further include an internal memory (720), the pollution information collection unit (600) may include at least any one among a fine dust sensor, a CO₂ sensor, and a volatile organic compounds (VOC) sensor, the control unit (700) may further include a signal processor (730) to compare a pollution critical value stored in the internal memory (720) and pollution information collected by the pollution information collection unit (600) so as to control a rotation speed and a rotation time of the driving motor (200), and the collected pollution information versus driving information of the driving motor (200) may be mapped and stored in the internal memory (720) in advance.

The signal processor (730) may generate a pollution trigger to drive a contamination processor when receiving the pollution information from the pollution information collection unit (600) and generate a control trigger to drive a control processor when receiving a control signal from the communication module (710), the contamination processor and the control processor may be provided to determine turning ON/OFF, a rotation speed, or a rotation time of the driving motor (200) according to priority information preset in the internal memory (720), and the control unit (700) may be provided to transmit determined information to an external device through the communication module (710) according to the determined information.

### BRIEF DESCRIPTION OF THE DRAWINGS

Since the accompanying drawings of the present specification illustrate exemplary embodiments and serve to convey the technical spirit of the present invention with the detailed description of the present invention, the present invention should not be limited and interpreted by contents in the accompanying drawings, in which:
FIGS. 1 and 2 are views for describing an insert fan of an air cleaner according to one exemplary embodiment of the present invention;
FIG. 3 is a three-dimensional (3D) view for describing an accommodation space of the air cleaner according to one exemplary embodiment of the present invention;
FIGS. 4 and 5 are three-dimensional views for describing a cover member of the air cleaner according to one exemplary embodiment of the present invention;
FIGS. 6 and 14 are 3D views for describing an ultraviolet (UV) lamp of the air cleaner according to one exemplary embodiment of the present invention;
FIGS. 7 to 9 are views for describing a filter front cover of the air cleaner according to one exemplary embodiment of the present invention;
FIGS. 10 and 11 are 3D views for describing a filter member of the air cleaner according to one exemplary embodiment of the present invention;
FIGS. 12 and 13 are views for describing a filter rear cover of the air cleaner according to one exemplary embodiment of the present invention; and
FIGS. 15 and 16 are views for describing a structure of the air cleaner according to one exemplary embodiment of the present invention.

### DETAILED DESCRIPTION OF EXEMPLARY EMBODIMENTS

In embodiments according to the concept of the present invention disclosed in the specification, specific structural and functional descriptions are directed only to providing examples for describing the embodiments of the present invention, and the embodiments according to the concept of the present invention may be implemented in various forms, and thus the present invention is not limited by the embodiments described in the specification.

Since the embodiments according to the concept of the present invention may be variously modified and have various forms, the embodiments will be illustrated in the drawings and described in detail in the present specification. However, there is no intent to limit the present invention to the particular forms disclosed, and the present invention covers all modifications, equivalents, and alternatives falling within the spirit and scope of the present invention.

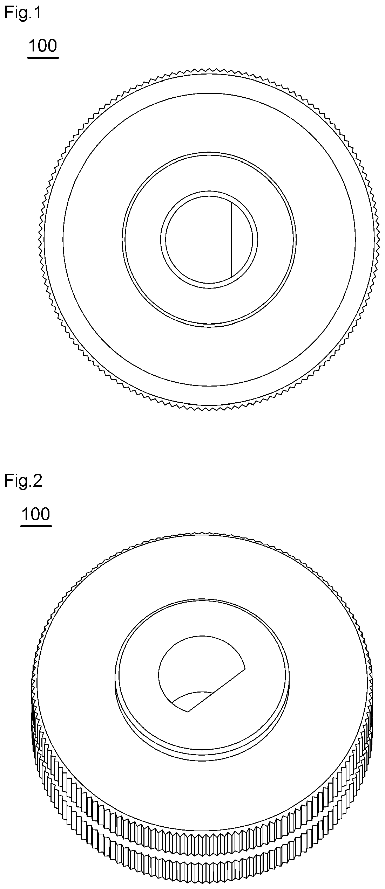

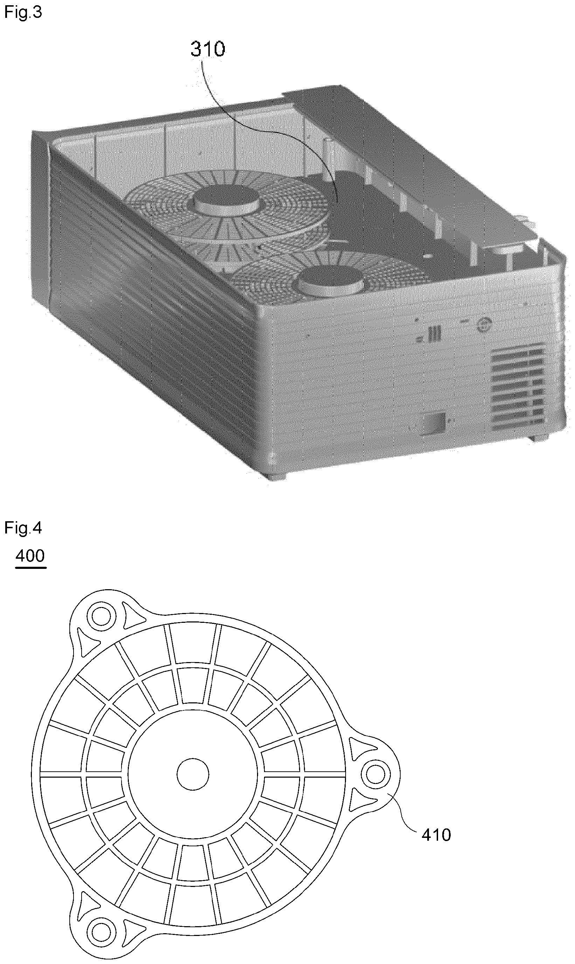

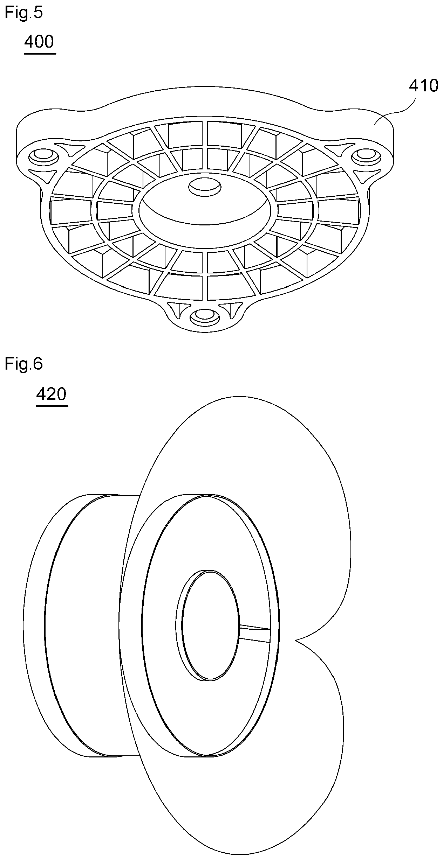

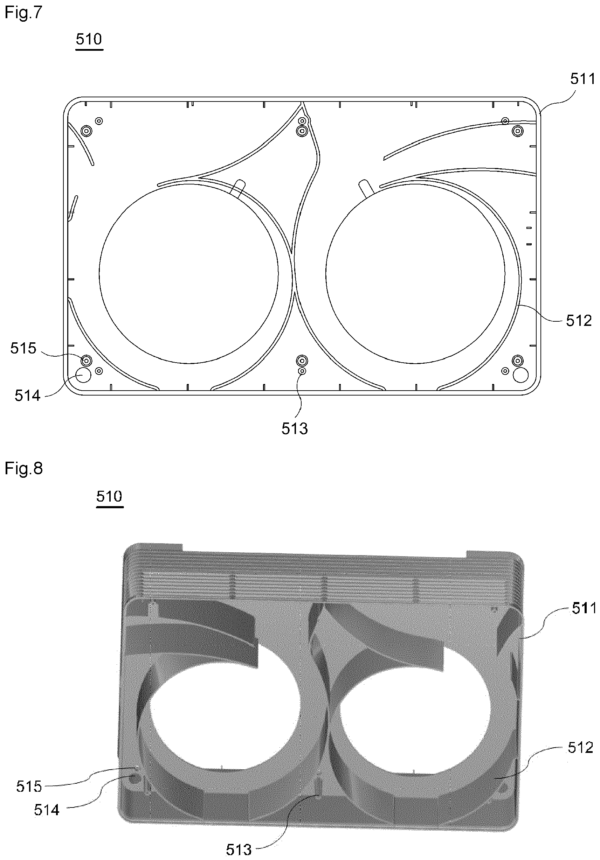

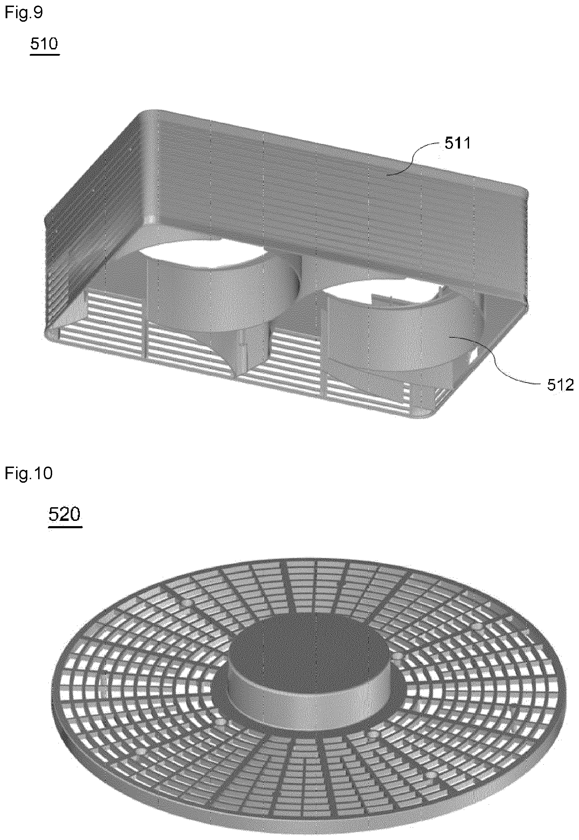

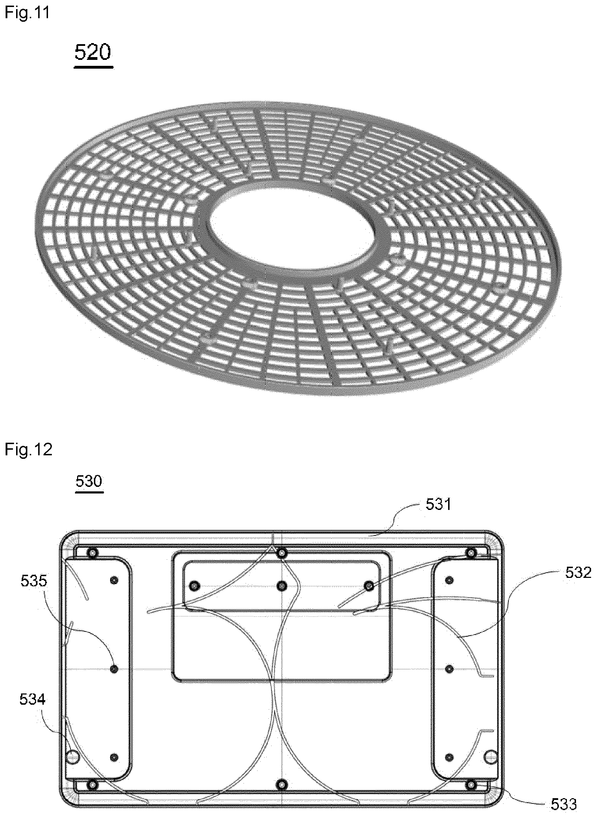

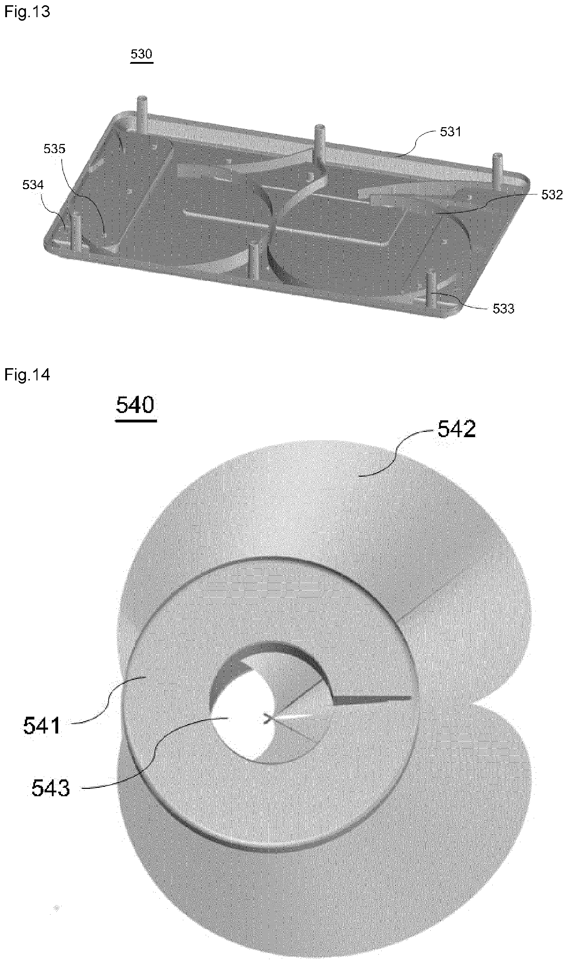

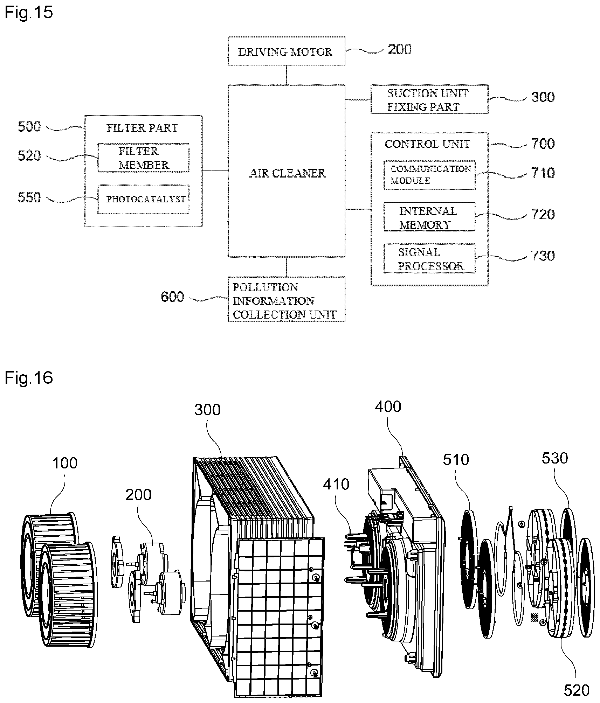

FIGS. 1 and 2 are views for describing an insert fan of an air cleaner according to one exemplary embodiment of the present invention, FIG. 3 is a three-dimensional (3D) view for describing an accommodation space of the air cleaner according to one exemplary embodiment of the present invention, FIGS. 4 and 5 are 3D views for describing a cover member of the air cleaner according to one exemplary embodiment of the present invention, FIGS. 6 and 14 are 3D views for describing an ultraviolet (UV) lamp of the air cleaner according to one exemplary embodiment of the present invention, FIGS. 7 to 9 are views for describing a filter front cover of the air cleaner according to one exemplary embodiment of the present invention, FIGS. 10 and 11 are 3D views for describing a filter member of the air cleaner according to one exemplary embodiment of the present invention, FIGS. 12 and 13 are views for describing a filter rear cover of the air cleaner according to one exemplary embodiment of the present invention, and FIG. 16 is a view for describing a configuration of the air cleaner according to one exemplary embodiment of the present invention.

First, as illustrated in FIGS. 1, 2, and 16, an air cleaner according to one embodiment of the present invention includes an insert fan 100 which is disposed inward from a suction port of a casing forming an exterior and including the suction port disposed at one side and a discharge port disposed at the other side, and suctions ambient air.

In addition, a box for surrounding an outer portion of the insert fan 100 may be formed.

As illustrated in FIG. 15, a driving motor 200 provided to be axially detachably coupled to the insert fan 100 in a direction from the discharge port and a suction unit fixing part 300 in which an accommodation space 310, into which the insert fan 100 and the driving motor 200 are fixedly inserted in the direction from the suction port toward the discharge port and which is finished by inserting a cover member 400 in the direction from the discharge port, are provided.

In this case, as illustrated in FIGS. 4 to 6, the cover member 400 is provided so that a plurality of protruding fixing members 410 are disposed on a side surface of the suction unit fixing part 300 to fix the driving motor 200 in the direction from the discharge port and a plurality of first UV lamps 420 are disposed to provide light emission toward the suction port.

In addition, as illustrated in FIG. 15, a filter part 500 which is provided to be pressed against and coupled to the cover member 400 in the direction from the discharge port and purifies air discharged by the insert fan 100, a pollution information collection unit 600, and a control unit 700 which transmits pollution information collected by the pollution information collection unit 600 to an external device through a communication module 710 and controls the driving motor 200, the first UV lamps 420, and the filter part 500 using a control signal received from an external device or an input control signal are provided.

In this case, as illustrated in FIGS. 8 to 13, a filter front cover 510, a filter member 520, and a filter rear cover 530 are sequentially disposed in a direction from the insert fan 100 toward the discharge port. The filter front cover 510 is provided with a first edge 511 having a circular shape and a protruding fixing member 512 having a circular shape and disposed at an inner center of the first edge 511, first support ribs 513 radially formed between the first edge 511 and the protruding fixing member 512, a plurality of first air through holes 514 formed between the first support ribs 513, and a plurality of first fixing through holes 515 disposed in the first support rib 513 to fix the filter front cover 510 and the filter rear cover 530. The filter rear cover 530 is provided with a second edge 531 having the same size and the same shape as the first edge 511, an accommodation edge 532, into which the protruding fixing member 512 is fixedly inserted, formed at a central position of the filter rear cover 530, a second support rib 533 and a plurality of second air through holes 534 formed between the second edge 531 and the accommodation edge 532, and second fixing through holes 535 are further provided at positions corresponding to the first fixing through holes 515.

In addition, as illustrated in FIG. 14, a second UV lamp 540 and a photocatalyst 550 are further included between the filter front cover 510 and the filter member 520, the second UV lamp 540 includes a lamp support 541 having a lamp shade 542 to maintain an angle at which light is emitted from the filter front cover 510 toward the filter member 520, the second UV lamp 540 is fixed to the lamp support 541, lengths of left and right sides of the lamp shade 542 are asymmetrically formed to be different, and one side having a greater length is formed as a shade space part 543 so that UV light being emitted is directly emitted to the filter member 520.

In this case, a surface of a base material having a spherical shape is coated with a photocatalyst agent, wherein the base material is prepared by mixing and grinding a phosphorescent material at 37 wt%, a zeolite at 37 wt%, a binder (sodium silicate) at 25 wt%, a titanium dioxide at 1 wt%, and ceramic balls to prepare a base powder material, applying moisture to the prepared base powder material to prepare the base powder material in a dough state, and balling and drying the base powder material in the dough state, and the photocatalyst agent is an agent in which any one oxide among a titanium dioxide (TiO₂), a zirconia (ZrO₂), a zinc oxide (ZnO), an iron dioxide (Fe₂O₃) is dispersed in a solvent.

In addition, a pair of left and right insert fans 100, a pair of left and right driving motors 200, and a pair of left and right filter parts 500 are each provided as one group, the accommodation space 310 is disposed to be divided into left and right spaces side by side so that the suction unit fixing part 300 accommodates the insert fans 100 and the driving motors 200 each disposed in the pair side by side, and the suction unit fixing part 300 further includes an air guide formed spirally in a rotation direction of the insert fans 100 and the driving motors 200 therein.

In this case, as illustrated in FIG. 15, the control unit 700 further includes an internal memory 720, the pollution information collection unit 600 includes at least any one among a fine dust sensor, a CO₂ sensor, and a volatile organic compounds (VOC) sensor, and the control unit 700 further includes a signal processor 730 to compare a pollution critical value stored in the internal memory 720 and pollution information collected by the pollution information collection unit 600 so as to control a rotation speed and a rotation time of the driving motor 200, and the collected pollution information versus driving information of the driving motor 200 are mapped and stored in the internal memory 720 in advance.

In this case, the signal processor 730 generates a pollution trigger to drive a contamination processor when receiving the pollution information from the pollution information collection unit 600 and generates a control trigger to drive a control processor when receiving a control signal through the communication module 710, the contamination processor and the control processor are provided to determine turning ON/OFF, a rotation speed, or a rotation time of the driving motor 200 according to priority information preset in the internal memory 720, and the control unit 700 transmits determined information to an external device through the communication module 710 according to the determined information.

When the air cleaner according to one embodiment of the present invention described above is used, there are effects as follows. Outside air is suctioned using the insert fan 100 which is disposed at the inner side in the direction from the suction port of the casing forming the exterior and including the suction port disposed at one side and the discharge port disposed at the other side, the driving motor 200 provided to be axially detachably coupled to the insert fan 100 in the direction from the discharge port, and the suction unit fixing part 300 in which the accommodation space 310, into which the insert fan 100 and the driving motor 200 are fixedly inserted in the direction from the suction port toward the discharge port and which is finished by inserting the cover member 400 in the direction from the discharge port. The driving motor is stably fixed and a direction, in which light is emitted, is set to facilitate filtering through the cover member 400 is provided so that the plurality of protruding fixing members 410 are disposed on the side surface of the suction unit fixing part 300 to fix the driving motor 200 in the direction from the discharge port and the plurality of first UV lamps 420 are disposed to provide light emission toward the suction port. Polluted air is purified, pollution information is collected, and the air cleaner is easily controlled according to a degree of pollution by the filter part 500 which is provided to be pressed against and coupled to the cover member 400 in the direction from the discharge port and purifies air discharged by the insert fan 100, the pollution information collection unit 600, and the control unit 700 which transmits pollution information collected by the pollution information collection unit 600 to an external device through the communication module 710 and controls the driving motor 200, the first UV lamps 420, and the filter part 500 using a control signal received from an external device or an input control signal. A support force of a cover attached to a filter is improved and air easily flows by sequentially arranging the filter front cover 510, the filter member 520, and the filter rear cover 530 in the direction from the insert fan 100 toward the discharge port, wherein the filter front cover 510 is provided with the first edge 511 having a circular shape and the protruding fixing member 512 having a circular shape and disposed at the inner center of the first edge 511, the first support ribs 513 radially formed between the first edge 511 and the protruding fixing member 512, the plurality of first air through holes 514 formed between the first support ribs 513, the plurality of first fixing through holes 515 disposed in the first support rib 513 to fix the filter front cover 510 and the filter rear cover 530. A support force is improved and air circulation is easily accomplished and includes the filter rear cover (530) provided with the second edge 531 having the same size and the same shape as the first edge 511, the accommodation edge 532, into which the protruding fixing member 512 are fixedly inserted, formed at a central position thereof, the second support rib 533 and the plurality of second air through holes 534 formed between the second edge 531 and the accommodation edge 532, and the second fixing through holes 535 further provided at positions corresponding to the first fixing through holes 515. There are effects of decomposing pollutants using a photocatalyst and easily fixing the UV lamp by further including the second UV lamp 540 and the photocatalyst 550 between the filter front cover 510 and the filter member 520, wherein the lamp support 541 having the lamp shade 542 to maintain an angle at which light is emitted in the direction from the filter front cover 510 toward the filter member 520 is provided, the second UV lamp 540 is fixed to the lamp support 541, the lengths of left and right sides of the lamp shade 542 are asymmetrically formed to be different, and one side having a greater length is formed as the shade space part 543 so that UV light being emitted is directly emitted to the filter member 520. Harmful substances may be decomposed through an oxidation reaction by coating the surface of the photocatalyst 550 with the photocatalyst agent, wherein the base material is prepared by mixing and grinding the phosphorescent material at 37 wt%, the zeolite at 37 wt%, the binder (sodium silicate) at 25 wt%, the titanium dioxide at 1 wt%, and the ceramic balls to prepare the base powder material, applying moisture to the prepared base powder material to prepare the base powder material in a dough state, and balling and drying the base powder material in the dough state, and the photocatalyst agent is the agent in which any one oxide among the titanium dioxide (TiO₂), the zirconia (ZrO₂), the zinc oxide (ZnO), and the iron dioxide (Fe₂O₃) is dispersed in a solvent. An air flow may be controlled to facilitate filtering by providing the pair of left and right insert fans 100, the pair of left and right driving motors 200, and the pair of left and right filter parts 500 each provided as one group, wherein the accommodation space 310 is disposed to be divided into left and right spaces side by side so that the suction unit fixing part 300 accommodates the insert fans 100 and the driving motors 200 each disposed in the pair side by side, and the suction unit fixing part 300 further includes the air guide formed spirally in the rotation direction of the insert fan 100 and the driving motor 200 therein. The control unit 700 further includes the internal memory 720, and the pollution information collection unit 600 includes at least any one among the fine dust sensor, the CO₂ sensor, and the volatile organic compounds (VOC) sensor, wherein the signal processor 730, which compares a pollution critical value stored in the internal memory 720 and pollution information collected by the pollution information collection unit 600 so as to control a rotation speed and a rotation time of the driving motor 200, is further provided, and the collected pollution information versus driving information of the driving motor 200 are mapped and stored in the internal memory 720 in advance.

Control is easily performed from an external device through the communication module by generating, by the signal processor 730, a pollution trigger to drive the contamination processor when receiving the pollution information from the pollution information collection unit 600, generating, by the signal processor 730, a control trigger to drive the control processor when receiving a control signal through the communication module 710, wherein the contamination processor and the control processor are provided to determine turning ON/OFF, a rotation speed, or a rotation time of the driving motor 200 according to priority information preset in the internal memory 720, and transmitting determined information to an external device through the communication module 710 according to the information determined by the control unit 700.

The present invention provides an air cleaner and achieves the following effects.

First, there is an effect of suctioning air using an insert fan 100 which is disposed inward from a suction port of a casing forming an exterior and including the suction port disposed at one side and a discharge port disposed at the other side, a driving motor 200 provided to be axially detachably coupled to the insert fan 100 in a direction from the discharge port, and a suction unit fixing part 300 in which an accommodation space 310, into which the insert fan 100 and the driving motor 200 are fixedly inserted in the direction from the suction port toward the discharge port and which is finished by inserting a cover member 400 in the direction from the discharge port.

Second, there are effects of stably fixing the driving motor and setting a direction, in which light is emitted, to facilitate filtering through the cover member 400 which is provided so that a plurality of protruding fixing members 410 are disposed on a side surface of the suction unit fixing part 300 to fix the driving motor 200 in the direction from the discharge port and a plurality of first UV lamps 420 are disposed to provide light emission toward the suction port.

Third, polluted air can be purified, pollution information can be collected, and the air cleaner can be easily controlled according to a degree of pollution by a filter part 500 which is provided to be pressed against and coupled to the cover member 400 in the direction from the discharge port and purifies air discharged by the insert fan 100, a pollution information collection unit 600, and a control unit 700 which transmits pollution information collected by the pollution information collection unit 600 to an external device through a communication module 710 and controls the driving motor 200, the first UV lamps 420, and the filter part 500 using a control signal received from an external device or an input control signal.

Fourth, there are effects of improving a support force of a cover attached to a filter and allowing air to easily flow by sequentially arranging a filter front cover 510, a filter member 520, and a filter rear cover 530 in the direction from the insert fans 100 toward the discharge port, wherein the filter front cover 510 is provided with a first edge 511 having a circular shape and a protruding fixing member 512 having a circular shape and disposed at an inner center of the first edge 511, first support ribs 513 radially formed between the first edge 511 and the protruding fixing member 512, a plurality of first air through holes 514 formed between the first support ribs 513, and a plurality of first fixing through holes 515 disposed in the first support rib 513 to fix the filter front cover 510 and the filter rear cover 530.

Fifth, a support force can be improved and air circulation can be easily accomplished by forming a second edge 531 having the same size and the same shape as the first edge 511, an accommodation edge 532, into which the protruding fixing member 512 is fixedly inserted at a central position, and a second support rib 533 and a plurality of second air through holes 534 between the second edge 531 and the accommodation edge 532, and further providing second fixing through holes 535 at positions corresponding to the first fixing through holes 515.

Sixth, there are effects of decomposing pollutants using a photocatalyst and easily fixing a UV lamp by further including a second UV lamp 540 and a photocatalyst 550 between the filter front cover 510 and the filter member 520, wherein a lamp support 541 having a lamp shade 542 to maintain an angle at which light is emitted in a direction from the filter front cover 510 toward the filter member 520 is provided, the second UV lamp 540 is fixed to the lamp support 541, lengths of left and right sides of the lamp shade 542 are asymmetrically formed to be different, and one side having a greater length is formed as a shade space part 543 so that UV light being emitted is directly emitted to the filter member 520.

Seventh, harmful substances can be decomposed through an oxidation reaction by coating a surface of the photocatalyst 550 with a photocatalyst agent, wherein the base material is prepared by mixing and grinding a phosphorescent material at 37 wt%, a zeolite at 37 wt%, a binder (sodium silicate) at 25 wt%, a titanium dioxide at 1 wt%, and ceramic balls to prepare a base powder material, applying moisture to the prepared base powder material to prepare the base powder material in a dough state, and balling and drying the base powder material in the dough state, and the photocatalyst agent is an agent in which any one oxide among a titanium dioxide (TiO₂), a zirconia (ZrO₂), a zinc oxide (ZnO), and an iron dioxide (Fe₂O₃) is dispersed in a solvent.

Eighth, an air flow can be controlled to facilitate filtering by providing a pair of left and right insert fans 100, a pair of left and right driving motors 200, and a pair of left and right filter parts 500 each provided as one group, wherein the accommodation space 310 is disposed to be divided into left and right spaces side by side so that the suction unit fixing part 300 accommodates the insert fans 100 and the driving motors 200 each disposed in the pair side by side, and the suction unit fixing part 300 further includes an air guide formed spirally in a rotation direction of the insert fan 100 and the driving motor 200 therein.

Nineth, the control unit 700 further includes an internal memory 720, and the pollution information collection unit 600 includes at least any one among a fine dust sensor, a CO₂ sensor, and a volatile organic compounds (VOC) sensor in, wherein a signal processor 730, which compares a pollution critical value stored in the internal memory 720 and pollution information collected by the pollution information collection unit 600 so as to control a rotation speed and a rotation time of the driving motors 200, is further provided, and the collected pollution information versus driving information of the driving motor 200 can be mapped and stored in the internal memory 720 in advance.

Tenth, there is an effect that the air cleaner can be easily controlled from an external device through a communication module by generating, by the signal processor 730, a pollution trigger to drive a contamination processor when receiving the pollution information from the pollution information collection unit 600, generating, by the signal processor 730, a control trigger to drive a control processor when receiving a control signal through the communication module 710, wherein the contamination processor and the control processor are provided to determine turning ON/OFF, a rotation speed, or a rotation time of the driving motor 200 according to priority information preset in the internal memory 720, and transmitting determined information to an external device through the communication module 710 according to the information determined by the control unit 700.

Meanwhile, effects to be achieved through the present invention are not limited to the above-described effects, and other effects which are not described above will be clearly understood by those skilled in the art through the following specification.

The present invention has been described with reference to exemplary embodiments as described above, but the exemplary embodiments are only examples describing the technical spirit of the present invention, and the fact that variously modification may be made without departing from the technical spirit of the present invention can be understood by those skilled in the art. Accordingly, the scope of protection of the present invention should be interpreted not by a specific embodiment but by content defined by the appended claims and encompass all equivalents falling within the technical spirit.

## Claims

1. An air cleaner comprising:
an insert fan (100) which is disposed inward from a suction port of a casing forming an exterior and including the suction port disposed at one side and a discharge port disposed at the other side, and suctions ambient air;
a driving motor (200) provided to be axially detachably coupled to the insert fan (100) in a direction from the discharge port;
a suction unit fixing part (300) in which an accommodation space (310), into which the insert fan (100) and the driving motor (200) are fixedly inserted in a direction from the suction port toward the discharge port and which is finished by inserting a cover member (400) in the direction from the discharge port;
the cover member (400) in which a plurality of protruding fixing members (410) are disposed on a side surface of the suction unit fixing part (300) to fix the driving motor (200) in the direction from the discharge port, and a plurality of first ultraviolet (UV) lamps (420) are disposed to provide light emission toward the suction port;
a filter part (500) which is provided to be pressed against and coupled to the cover member (400) in the direction from the discharge port and purifies air discharged by the insert fan (100);
a pollution information collection unit (600) disposed in or around the casing to collect information about surrounding air; and
a control unit (700) which transmits pollution information collected by the pollution information collection unit (600) to an outside through a communication module (710) and controls the driving motor (200), the first UV lamps (420), and the filter part (500) using a control signal received from an external device or an input control signal.

2. The air cleaner of claim 1, wherein the filter part (500) includes a filter front cover (510), a filter member (520), and a filter rear cover (530) that are sequentially disposed in a direction from the insert fan (100) toward the discharge port,
wherein the filter front cover (510) is provided with a first edge (511) having a circular shape and a protruding fixing member (512) having a circular shape and disposed at an inner center of the first edge (511),
first support ribs (513) radially formed between the first edge (511) and the protruding fixing member (512),
a plurality of first air through holes (514) formed between the first support ribs (513), and
a plurality of first fixing through holes (515) disposed in the first support rib (513) to fix the filter front cover (510) and the filter rear cover (530),
the filter rear cover (530) is provided with a second edge (531) having the same size and the same shape as the first edge (511),
an accommodation edge (532), into which the protruding fixing member (512) is fixedly inserted, formed at a central position thereof,
a second support rib (533) and a plurality of second air through holes (534) formed between the second edge (531) and the accommodation edge (532), and
second fixing through holes (535) further provided at positions corresponding to the first fixing through holes (515), and
the filter front cover (510), the filter member (520), and the filter rear cover (530) are provided as one module to be attachable to and detachable from the suction unit fixing part (300) and the cover member (400).

3. The air cleaner of claim 2, wherein the filter part (500) further includes a second UV lamp (540) and a photocatalyst (550) between the filter front cover (510) and the filter member (520),
wherein the second UV lamp (540) includes a lamp support (541) having a lamp shade (542) to maintain an angle at which light is emitted from the filter front cover (510) toward the filter member (520),
the second UV lamp (540) is fixed to the lamp support (541),
lengths of left and right sides of the lamp shade (542) are asymmetrically formed to be different,
one side having a greater length is formed as a shade space part (543), and
UV light being emitted is directly emitted to the filter member (520).

4. The air cleaner of claim 1, wherein:
a pair of left and right insert fans (100), a pair of left and right driving motors (200), and a pair of left and right filter parts (500) are each provided as one group;
the accommodation space (310) is disposed to be divided into left and right spaces side by side so that the suction unit fixing part (300) accommodates the insert fans (100) and the driving motors (200) each disposed in the pair side by side;
the suction unit fixing part (300) further includes an air guide formed spirally in a rotation direction of the insert fans (100) and the driving motors (200) therein;
power supplied to the driving motors (200) is provided from independent circuits; and
even when any one driving motor (200) is turned OFF, the other driving motor (200) maintains a turned ON state.

5. The air cleaner of claim 1, wherein:
the control unit (700) further includes an internal memory (720);
the pollution information collection unit (600) includes at least any one among a fine dust sensor, a CO₂ sensor, and a volatile organic compounds (VOC) sensor;
the control unit (700) further includes a signal processor (730) to compare a pollution critical value stored in the internal memory (720) and pollution information collected by the pollution information collection unit (600) so as to control a rotation speed and a rotation time of the driving motor (200);
the collected pollution information versus driving information of the driving motor (200) are mapped and stored in the internal memory (720) in advance;
a total driving time of the driving motor (200) is stored in the internal memory (720);
when the total driving time is greater than a preset time, a replacement signal for the filter part (500) is transmitted to an external device through the communication module (710);
the signal processor (730) generates a pollution trigger to drive a contamination processor when receiving the pollution information from the pollution information collection unit (600) and generates a control trigger to drive a control processor when receiving a control signal from the communication module (710);
the contamination processor and the control processor are provided to determine turning ON/OFF, a rotation speed, or a rotation time of the driving motor (200) according to priority information preset in the internal memory (720); and
the control unit (700) is provided to transmit information determined according to the preset priority information to an external device through the communication module (710).
